# EUROPÄISCHE PATENTSCHRIFT

(11) **EP 3 672 692 B1**
(45) Veröffentlichungstag und Bekanntmachung des Hinweises auf die Patenterteilung: **16.08.2023**
(21) Anmeldenummer: 17758517.1
(22) Anmeldetag: 25.08.2017
(51) Int. Cl.: A61Q 13/00, C07D 317/70, C11B 9/00, A61Q 5/00, A61K 8/37, A61K 8/92

(54) **MISCHUNGEN ENTHALTEND ENANTIOMERENREINES AMBROCENIDE®**
BLENDS CONTAINING ENANTIOMERICALLY PURE AMBROCENIDE®
MÉLANGES CONTENANT DE L'AMBROCENIDE® ÉNANTIOMÉRIQUEMENT PUR

(43) Veröffentlichungstag der Anmeldung: 01.07.2020
(73) Patentinhaber: Symrise AG, 37603 Holzminden (DE)
(72) Erfinder: EH, Marcus, 37603 Holzminden (DE); LAMBRECHT, Stefan, 37619 Hehlen (DE)
(74) Vertreter: Eisenführ Speiser
(86) Internationale Anmeldenummer: PCT/EP2017/071424
(87) Internationale Veröffentlichungsnummer: WO 2017/186973

(56) Entgegenhaltungen:
- EP-A1- 1 947 166
- EP-A1- 2 947 078
- EP-A2- 1 634 864
- PANTEN J ET AL: "New woody and ambery notes from cedarwood and turpentine oil", CHEMISTRY & BIODIVERSITY, HELVETICA CHIMICA ACTA, ZUERICH, CH, Bd. 1, Nr. 12, 1. Dezember 2004 (2004-12-01), Seiten 1936-1948, XP002543298, ISSN: 1612-1872, DOI: 10.1002/CBDV.200490148

## Beschreibung

Die vorliegende Erfindung betrifft primär eine Mischung umfassend die Verbindung der Formel (la) wie hierin beschrieben, wobei die Mischung weniger als 0,01 Gew.-%, bezogen auf das Gesamtgewicht der Mischung, an Verbindung der Formel (Ib) wie hierin beschrieben, vorzugsweise an den Verbindungen der Formeln (Ib) sowie zudem (Ic) und/oder (Id) wie hierin beschrieben, enthält. Die Erfindung betrifft zudem ein Verfahren zur Herstellung besagter Mischung, Riechstoffkompositionen enthaltend oder bestehend aus besagter Mischung und einem oder mehreren zusätzlichen Riechstoffen, parfümierte Produkte enthaltend besagte Mischungen bzw. Riechstoffkompositionen, sowie diverse Verfahren und Verwendungen zum Vermitteln, Modifizieren und/oder Verstärken bestimmter Geruchsnoten.

Weitere Aspekte und bevorzugte Ausgestaltungen der vorliegenden Erfindung ergeben sich aus den nachfolgenden Ausführungen, den beigefügten Beispielen und insbesondere den beigefügten Patentansprüchen.

Ambrocenide^{®} besitzt die folgende chemische Struktur:

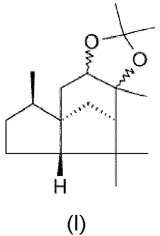

Die geschlängelten Linien können dabei unabhängig voneinander alpha- oder beta-Konfiguration bedeuten. Dementsprechend kann Ambrocenide^{®} generell ein, zwei, drei oder sämtliche der folgenden Stereoisomere umfassen:

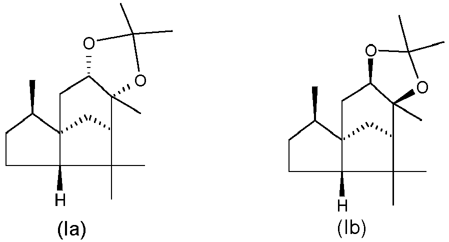

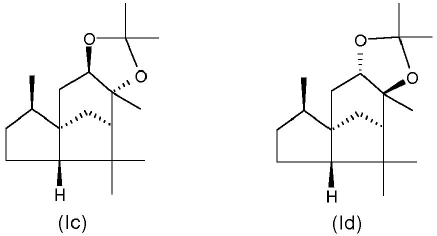

Eine Möglichkeit zur Herstellung von Ambrocenide^{®} wird in EP 0 857 723 B1 offenbart. Zunächst wird dabei (-)-alpha-Cedren (**1**) durch Behandeln mit Peressigsäure zu (-)-alpha-Cedrenepoxid (2) umgesetzt. Das erhaltene Epoxid (**2**) wird dann durch Säure katalysierte Ringöffnung in ein Gemisch der epimeren Cedran-Diole (**3**) übergeführt. Ambrocenide^{®} ((**4**) mit R=R'=CH₃: Verbindung der Formel (I)) kann anschließend aus den Diolen (**3**) durch Umsetzung mit Dimethoxypropan unter Säurekatalyse erhalten werden:

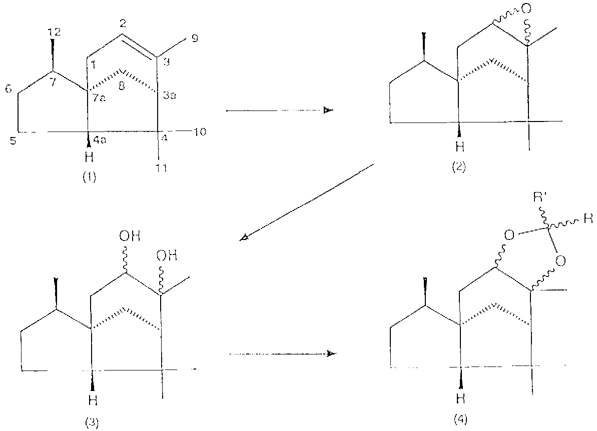

In der Offenlegungsschrift EP 2 947 078 A1 wird eine Mischung offenbart, welche die Verbindung der Formel (la) (wie hierin vorangehend beschrieben) und die Verbindung der Formel (Ib) (wie hierin vorangehend beschrieben) in einem Gewichtsverhältnis von 90 : 10 bis 99 : 1 umfasst. Eine solche Mischung kann in im Wesentlichen kristalliner Form bereitgestellt werden.

EP 1 947 166 A1 beschreibt Mischungen umfassend oder bestehend aus 3-(4-Methyl-cyclohex-3-enyl)-butyraldehyd (Limonenal) sowie Ambrocenide^{®}, wobei das Gewichtsverhältnis von Limonenal zu Ambrocenide^{®} im Bereich von 150 : 1 bis 2500 : 1 liegt.

In Abhängigkeit von den gewählten Reaktionsbedingungen liegt Ambrocenide^{®} jeweils als ein bestimmtes Diastereomerengemisch, d.h. als Mischung enthaltend zwei, drei oder sämtliche der Verbindungen der Formel (la), (Ib), (Ic) und (Id) (s. vorangehend dargestellte Strukturformeln) vor.

Eine Aufgabe der vorliegenden Erfindung war die Optimierung von Ambrocenide^{®} und die Bereitstellung vorzugsweise besonders stark geruchlich aktiver Mischungen, d.h. solcher Mischungen, die in der Lage sind, angenehme geruchliche Eigenschaften besonders effektiv zu vermitteln und/oder zu verstärken bzw. unangenehme Geruchseindrücke besonders effektiv zu maskieren und/oder zu vermindern.

Die obige Aufgabe wird gemäß einem ersten Aspekt der vorliegenden Erfindung gelöst durch eine Mischung umfassend die Verbindung der Formel (la), 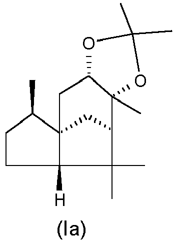 wobei die Mischung weniger als 0,01 Gew.-%, bezogen auf das Gesamtgewicht der Mischung, an Verbindung der Formel (Ib), vorzugsweise an den Verbindungen der Formeln (Ib) sowie zudem (Ic) und/oder (Id), enthält

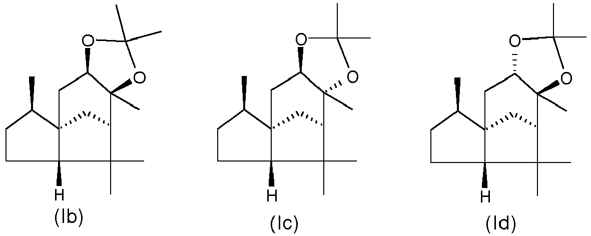

Im Rahmen der der vorliegenden Erfindung zugrunde liegenden Untersuchungen wurde überraschenderweise festgestellt, dass die Verbindung der Formel (la), wie hierin beschrieben, wesentlich stärker geruchlich aktiv ist als die Verbindungen der Formel (Ib), (Ic) und (Id). Die Verbindung der Formel (la) ist also in besonderem Maße für die geruchlichen Eigenschaften von Ambrocenide^{®} verantwortlich. Es war bisher unbekannt, welche der Stereoisomere von Ambrocenide^{®} welche geruchlichen Eigenschaften aufweisen.

Die erfindungsgemäße Mischung weist daher besonders vorteilhafte geruchliche Eigenschaften auf. Insbesondere ist es mit Hilfe der erfindungsgemäßen Mischung möglich, mit einer geringeren Konzentration an erfindungsgemäßer Mischung im Vergleich zu den im Stand der Technik bekannten Diastereomerengemischen von Ambrocenide^{®} die gleichen oder verbesserte Effekte in Bezug auf das Verstärken bzw. Vermitteln eines angenehmen Geruchseindrucks und/oder das Maskieren bzw. Vermindern eines unangenehmen Geruchseindrucks zu erzielen. Ein Vorteil der erfindungsgemäßen Mischung ist daher auch ihre hohe Duftintensität bei vergleichsweise geringer Dosierung. Dies ist aus Gründen der Umweltreinhaltung von besonderem Interesse, da hierdurch die Menge der in die Umwelt freigesetzten Stoffe minimal gehalten werden kann.

Ferner betrifft die vorliegende Erfindung gemäß einer bevorzugten Ausführungsform eine Mischung wie hierin beschrieben, zusätzlich enthaltend eine oder mehrere Verbindung(en) der Formel (II) 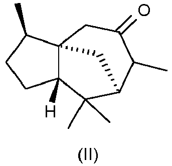 und/oder der Formel (III) 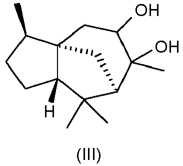 und/oder der Formel (IV) 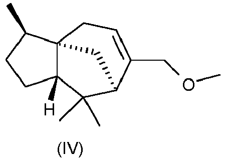 und/oder der Formel (V) 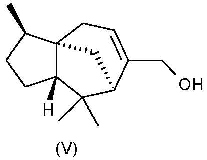 und/oder der Formel (VI)

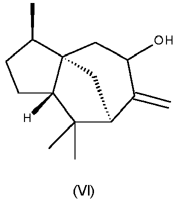

Insbesondere das Vorhandensein von Verbindung(en) der Formel (II) (Cedralon) in der erfindungsgemäßen Mischung ist besonders vorteilhaft, da es die Kopfnote verstärkt und den Geruch von Ambrocenide^{®} animalischer erscheinen lässt.

Besonders bevorzugt ist dabei eine Mischung wie hierin beschrieben, wobei die Mischung eine oder mehrere Verbindung(en) der Formel (II) enthält, 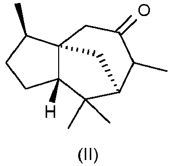
wobei das Gewichtsverhältnis der Gesamtmenge an Verbindung der Formel (la) zur Gesamtmenge an Verbindung(en) der Formel (II) in der Mischung 500 : 1 bis 3 : 1, vorzugsweise 350 : 1 bis 5 : 1, besonders bevorzugt 300 : 1 bis 8 : 1, beträgt,
und/oder wobei die Mischung eine oder mehrere Verbindung(en) der Formel (III) enthält, 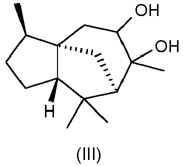
wobei das Gewichtsverhältnis der Gesamtmenge an Verbindung der Formel (la) zur Gesamtmenge an Verbindung(en) der Formel (III) in der Mischung wenigstens 5 : 1, vorzugsweise wenigstens 10 : 1, besonders bevorzugt wenigstens 15 : 1, beträgt,
und/oder wobei die Mischung eine oder mehrere Verbindung(en) der Formel (IV) enthält, 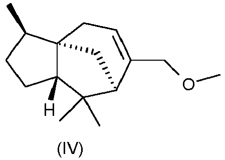
wobei das Gewichtsverhältnis der Gesamtmenge an Verbindung der Formel (la) zur Gesamtmenge an Verbindung(en) der Formel (IV) in der Mischung wenigstens 40 : 1, vorzugsweise wenigstens 50 : 1, besonders bevorzugt wenigstens 60 : 1, beträgt,
und/oder wobei die Mischung eine oder mehrere Verbindung(en) der Formel (V) enthält, 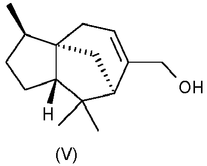
wobei das Gewichtsverhältnis der Gesamtmenge an Verbindung der Formel (la) zur Gesamtmenge an Verbindung(en) der Formel (V) in der Mischung wenigstens 30 : 1, vorzugsweise wenigstens 40 : 1, besonders bevorzugt wenigstens 50 : 1, beträgt,
und/oder wobei die Mischung eine oder mehrere Verbindung(en) der Formel (VI) enthält, 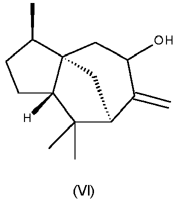
wobei das Gewichtsverhältnis der Gesamtmenge an Verbindung der Formel (la) zur Gesamtmenge an Verbindung(en) der Formel (VI) in der Mischung wenigstens 4 : 1, vorzugsweise wenigstens 6 : 1, besonders bevorzugt wenigstens 8 : 1, beträgt.

Besonders bevorzugt ist dabei eine Ausführungsform der erfindungsgemäßen Mischung, wobei die Mischung eine oder mehrere Verbindung(en) der Formel (II) wie hierin beschrieben enthält, und keine Verbindung(en) der Formel (III) bis (VI) wie hierin beschrieben enthält. Insbesondere bevorzugt ist dabei eine Ausführungsform der erfindungsgemäßen Mischung, wobei die Mischung eine Verbindung der Formel (II) wie hierin beschrieben enthält, und keine Verbindung(en) der Formel (III) bis (VI) wie hierin beschrieben enthält, und wobei das Gewichtsverhältnis der Gesamtmenge an Verbindung der Formel (la) zur Gesamtmenge an Verbindung(en) der Formel (II) in der Mischung 500 : 1 bis 3 : 1, vorzugsweise 350 : 1 bis 5 : 1, besonders bevorzugt 300 : 1 bis 8 : 1, insbesondere bevorzugt 280 : 1 bis 260 : 1, beträgt.

Des Weiteren bevorzugt ist eine alternative Ausführungsform der erfindungsgemäßen Mischung, wobei die Mischung jeweils zumindest eine oder mehrere Verbindung(en) der Formel (II), (III), (IV), (V) und (VI) wie hierin beschrieben enthält. Insbesondere bevorzugt ist dabei eine solche alternative Ausführungsform der erfindungsgemäßen Mischung, wobei die Mischung jeweils zumindest eine oder mehrere Verbindung(en) der Formel (II), (III), (IV), (V) und (VI) wie hierin beschrieben enthält, und wobei das Gewichtsverhältnis der Gesamtmenge an Verbindung der Formel (la) zur Gesamtmenge an Verbindung(en) der Formel (II) in der Mischung 500 : 1 bis 3 : 1, vorzugsweise 350 : 1 bis 5 : 1, besonders bevorzugt 300 : 1 bis 8 : 1, insbesondere bevorzugt 12 : 1 bis 8 : 1, beträgt.

Ein weiterer Aspekt der vorliegenden Erfindung betrifft ein Verfahren zur Herstellung einer Mischung wie hierin beschrieben, bestehend aus oder umfassend folgende Schritte:
a) Bereitstellen einer Ausgangsmischung, enthaltend oder im Wesentlichen bestehend aus alpha,alpha-Cedrandiol der Formel (Illa), 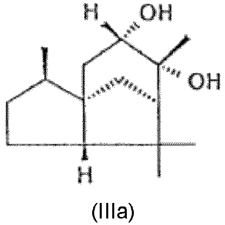 wobei die Ausgangsmischung weniger als 0,01 Gew.-%, bezogen auf das Gesamtgewicht der Ausgangsmischung, an beta,beta-Cedrandiol der Formel (Illb) enthält, 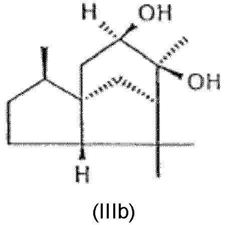 vorzugsweise wobei die Ausgangsmischung weniger als 0,01 Gew.-%, bezogen auf das Gesamtgewicht der Ausgangsmischung, an beta,beta-Cedrandiol der Formel (IIIb) sowie zudem von beta,alpha-Cedrandiol der Formel (IIIc) und/oder alpha,beta-Cedrandiol der Formel (IIId) enthält, 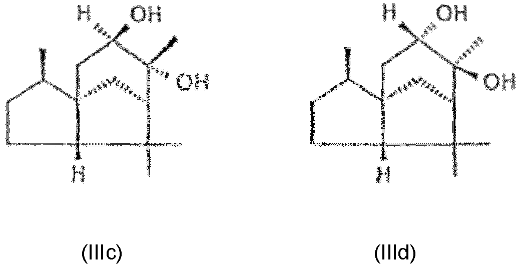 oder Bereitstellen der Verbindung alpha,alpha-Cedrandiol der Formel (Illa), 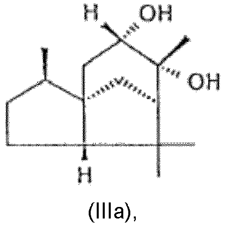
b) Umsetzen der Ausgangsmischung bzw. der Verbindung aus Schritt a) mit Dimethoxypropan,
wobei das Verfahren vorzugsweise zudem folgenden Schritt umfasst:
c) Auskristallisieren des Reaktionsprodukts aus Schritt b) aus wässriger alkoholischer Lösung.

Unter "im Wesentlichen bestehend aus alpha,alpha-Cedrandiol der Formel (Illa)" in Schritt a) des Verfahrens wird gemäß der vorliegenden Erfindung verstanden, dass die erfindungsgemäße Ausgangsmischung mindestens 95 Gew.-%, bevorzugt mindestens 98 Gew.-%, besonders bevorzugt mindestens 99 Gew.-%, ganz besonders bevorzugt mindestens 99,9 Gew.-%, weiter bevorzugt mindestens 99,99 Gew.%, bezogen auf das Gesamtgewicht der Ausgangsmischung, alpha,alpha-Cedrandiol der Formel (Illa) enthält.

Bezüglich des oben genannten Schrittes b) des erfindungsgemäßen Verfahrens ist ein Umsetzen der Ausgangsmischung bzw. der Verbindung aus Schritt a) mit Dimethoxypropan im Molverhältnis im Bereich von etwa 1 : 5 bis etwa 1 : 1, insbesondere von etwa 1 : 3, bezogen auf die Gesamt-Molmenge an Verbindungen der Formel (III) zur Gesamt-Molmenge an Dimethoxypropan, bevorzugt.

Ein weiterer Aspekt der vorliegenden Erfindung betrifft eine Mischung wie hierin beschrieben, herstellbar durch ein Verfahren wie hierin beschrieben.

Die vorangehend beschriebenen Definitionen und (bevorzugten) Ausführungsformen des erfindungsgemäßen Verfahrens sind dabei entsprechend anwendbar und führen zu besonders bevorzugten Ausführungsformen der erfindungsgemäßen Mischung.

In einem weiteren Aspekt betrifft die vorliegende Erfindung eine Riechstoffkomposition, vorzugsweise Parfümöl, enthaltend oder bestehend aus einer erfindungsgemäßen Mischung wie hierin beschrieben und zudem einem oder mehreren zusätzlichen Riechstoffen,
vorzugsweise wobei der zusätzliche bzw. einer, mehrere oder sämtliche der zusätzlichen Riechstoffe ausgewählt ist bzw. sind aus der Gruppe bestehend aus 3-(4-Methyl-1-cyclohex-3-enyl)-butanal, 4-(4-Hydroxyphenyl) butan-2-on, (E)-4-(2,6,6-Trimethyl-1-cyclohex-2-enyl)but-3-en-2-on, (E)-4-(2,6,6-Trimethyl-1-cyclohex-2-enyl)but-3-en-2-on, (E)-1-(2,6,6-trimethyl-cyclohexen-1-yl)pent-1-en-3-on, (E)-4-[(1S)-1,2,6,6-tetramethylcyclohex-2-en-1-yl]-but-3-en-2-on, 1-(2,6,6-Trimethyl-1-cyclohex-2-enyl)pent-1-en-3-on, [(Z)-Hex-3-enyl] methyl carbonat, 3-[(Z)-Hex-3-enoxy]propannitril, 1-(2,3,8,8-Tetramethyl-1,3,4,5,6,7-hexa-hydronaphthalin-2-yl)ethanon, spiro[1,3-dioxolan-2,5'-(4',4',8',8'-tetramethyl-hexahydro-3',9'-methanonaphthalin)], [3R-(3α,3aβ,6β,7β,8aα)]-octa-hydro-6-methoxy-3,6,8,8-tetramethyl-1H-3a,7-methanoazulen, [3R-(3α,3aβ,7β,8aα)]-1-(2,3,4,7,8,8a-hexahydro-3,6,8,8-tetramethyl-1H-3a,7-methano-azulen-5-yl)ethan-1-on, 1-(2,2,6-Trimethyl-cyclohexyl) hexan-3-ol, 6,6-Dimethoxy-2,5,5-trimethylhex-2-en, 2,6-Dimethyloct-7-en-2-ol, 3,7-Dimethylocta-1,6-dien-3-ol, (3,7-Dimethylocta-1,6-dien-3-yl)acetat, (4-Methyl-1-propan-2-yl-1-cyclohex-2-enyl)acetat, (8E)-Cyclohexadec-8-en-1-on, 16-Oxacyclohexa-decan-1-on, 1,3,4,6,7,8-Hexahydro-4,6,6,7,8,8-hexamethyl-cyclopenta(g)-2-benzopyran, Ethoxymethoxycyclo-dodecan, 1,1,2,3,3-Pentamethyl-2,5,6,7-tetrahydroinden-4-on, 1-(2,3,8,8-Tetramethyl-1,3,4,5,6,7-hexa-hydronaphthalin-2-yl)ethanon.

Ebenso betrifft die vorliegende Erfindung gemäß einer weiteren bevorzugten Ausführungsform auch eine Riechstoffkomposition wie hierin beschrieben, wobei die Menge an erfindungsgemäßer Mischung wie hierin beschrieben oder die Menge an Verbindung der Formel (la) ausreicht, 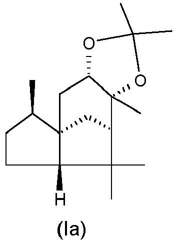
(a) um den bzw. einen oder mehrere unangenehme Geruchseindrücke eines anderen Riechstoffs in der Riechstoffkomposition zu maskieren oder zu vermindern,
   und/oder
(b) um den bzw. einen oder mehrere angenehme Geruchseindrücke eines anderen Riechstoffs in der Riechstoffkomposition zu verstärken.

Bevorzugt ist weiterhin eine erfindungsgemäße Riechstoffkomposition, vorzugsweise Parfümöl, wie hierin beschrieben, wobei die Gesamtmenge an Verbindung der Formel (la) 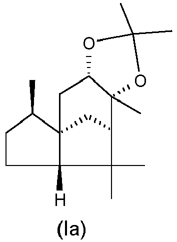 bezogen auf das Gesamtgewicht der Riechstoffkomposition 0,01 bis 10 Gew.-%, vorzugsweise 0,03 bis 5 Gew.-%, besonders bevorzugt 0,05 bis 2 Gew.-%, beträgt.

In einem weiteren Aspekt betrifft die vorliegende Erfindung ein parfümiertes Produkt, enthaltend eine erfindungsgemäße Mischung wie hierin beschrieben oder vorzugsweise eine erfindungsgemäße Riechstoffkomposition, vorzugsweise ein Parfümöl, wie hierin beschrieben in einer sensorisch wirksamen Menge, wobei der Anteil der Mischung bzw. der Riechstoffkomposition bezogen auf das Gesamtgewicht des Produktes vorzugsweise im Bereich von 0,01 bis 10 Gew.-%, bevorzugt 0,1 bis 5 Gew.-%, besonders bevorzugt 0,25 bis 3 Gew.-%, liegt.

Gemäß einer bevorzugten Ausführungsform ist das erfindungsgemäße parfümierte Produkt wie hierin beschrieben ausgewählt aus der Gruppe bestehend aus Parfüm-Extraits, Eau de Parfums, Eau de Toilettes, Rasierwässer, Eau de Colognes, Pre-shave-Produkte, Splash-Colognes, parfümierte Erfrischungstücher, saure, alkalische und neutrale Reinigungsmittel, Textilerfrischer, Bügelhilfen, flüssige Waschmittel, pulverförmige Waschmittel, Wä-schevorbehandlungsmittel, Wäscheweichspüler, Waschseifen, Waschtabletten, Desinfektionsmittel, Oberflächendesinfektionsmittel, Luftverbesserer, Aerosolsprays, Wachse und Polituren, Körperpflegemittel, Handcremes und -lotionen, Fußcremes und -lotionen, Enthaarungscremes und -lotionen, After-shave-Cremes und -lotionen, Bräunungscremes und -lotionen, Haarpflegeprodukten, Deodorantien und Antiperspirantien, Produkte der dekorativen Kosmetik, Kerzen, Lampenöle, Räucherstäbchen, Insektizide, Repellentien und Treibstoffe.

In einem weiteren Aspekt betrifft die vorliegende Erfindung zudem ein Verfahren zum Herstellen eines parfümierten Produktes, vorzugsweise eines Produktes wie hierin beschrieben, umfassend folgende Schritte:
i) Bereitstellen einer erfindungsgemäßen Mischung wie hierin beschrieben oder einer erfindungsgemäßen Riechstoffkomposition wie hierin beschrieben,
ii) Bereitstellen eines oder mehrerer weiterer Bestandteile des herzustellenden parfümierten Produktes, und
iii) Inkontaktbringen oder Mischen der in Schritt ii) bereitgestellten weiteren Bestandteile mit einer sensorisch wirksamen Menge der in Schritt i) bereitgestellten Bestandteile.

In einem weiteren Aspekt betrifft die vorliegende Erfindung die Verwendung einer erfindungsgemäßen Mischung wie hierin beschrieben oder einer erfindungsgemäßen Riechstoffkomposition wie hierin beschrieben
(a) zum Maskieren oder Vermindern des bzw. eines oder mehrerer unangenehmer Geruchseindrücke eines oder mehrerer unangenehm riechender Stoffe,
   und/oder
(b) zum Verstärken des bzw. eines oder mehrerer angenehmer Geruchseindrücke eines oder mehrerer angenehm riechender Stoffe.

Gemäß einer bevorzugten Ausführungsform betrifft die vorliegende Erfindung eine Verwendung wie hierin beschrieben in einer Zusammensetzung, die einen oder mehrere (weitere) angenehm und/oder unangenehm riechende Stoffe enthält, deren unangenehmer Geruchseindruck durch eine erfindungsgemäße Mischung wie hierin beschrieben oder eine erfindungsgemäße Riechstoffkomposition wie hierin beschrieben maskiert oder vermindert und/oder deren angenehmer Geruchseindruck durch eine erfindungsgemäße Mischung wie hierin beschrieben oder eine erfindungsgemäße Riechstoffkomposition wie hierin beschrieben verstärkt wird, vorzugsweise wobei diese(r) angenehm und/oder unangenehm riechende Stoff bzw. einer, mehrere oder sämtliche dieser angenehm und/oder unangenehm riechenden Stoffe ausgewählt ist bzw. sind aus der Gruppe bestehend aus 3-(4-Methyl-1-cyclohex-3-enyl)-butanal, 4-(4-Hydroxyphenyl) butan-2-on, (E)-4-(2,6,6-Trimethyl-1-cyclohex-2-enyl)but-3-en-2-on, (E)-4-(2,6,6-Trimethyl-1-cyclohex-2-enyl)but-3-en-2-on, (E)-1-(2,6,6-trimethyl-cyclohexen-1-yl)pent-1-en-3-on, (E)-4-[(1S)-1,2,6,6-tetramethylcyclohex-2-en-1-yl]-but-3-en-2-on, 1-(2,6,6-Trimethyl-1-cyclohex-2-enyl)pent-1-en-3-on, [(Z)-Hex-3-enyl] methyl carbonat, 3-[(Z)-Hex-3-enoxy]propannitril, 1-(2,3,8,8-Tetramethyl-1,3,4,5,6,7-hexa-hydronaphthalin-2-yl)ethanon, spiro[1,3-dioxolan-2,5'-(4',4',8',8'-tetramethyl-hexahydro-3',9'-methano-naphthalin)], [3R-(3α,3aβ,6β,7β,8aα)]-octa-hydro-6-methoxy-3,6,8,8-tetramethyl-1H-3a,7-methanoazulen,[3R-(3α,3aβ,7β,8aα)]-1-(2,3,4,7,8,8a-hexa-hydro-3,6,8,8-tetramethyl-1H-3a,7-methano-azulen-5-yl)ethan-1-on, 1-(2,2,6-Trimethyl-cyclohexyl)hexan-3-ol, 6,6-Dimethoxy-2,5,5-trimethylhex-2-en, 2,6-Dimethyloct-7-en-2-ol, 3,7-Dimethylocta-1,6-dien-3-ol, (3,7-Dimethylocta-1,6-dien-3-yl)acetat, (4-Methyl-1-propan-2-yl-1-cyclohex-2-enyl) acetat, (8E)-Cyclohexadec-8-en-1-on, 16-Oxacyclohexa-decan-1-on, 1,3,4,6,7,8-Hexahydro-4,6,6,7,8,8-hexamethyl-cyclopenta(g)-2-benzopyran, Ethoxymethoxycyclododecan, 1,1,2,3,3-Pentamethyl-2,5,6,7-tetrahydroinden-4-on, 1-(2,3,8,8-Tetramethyl-1,3,4,5,6,7-hexa-hydronaphthalin-2-yl)ethanon.

Erfindungsgemäß bevorzugt ist eine Verwendung wie hierin beschrieben, zum Verstärken des bzw. eines oder mehrerer Geruchseindrücke ausgewählt aus der Gruppe bestehend aus den Geruchsnoten blumig, vorzugsweise Maiglöckchen, Amber, holzig, Moschus, Veilchen, Citrus und aldehydig.

In einem weiteren Aspekt betrifft die vorliegende Erfindung ein Verfahren
(a) zum Maskieren oder Vermindern des bzw. eines oder mehrerer unangenehmer Geruchseindrücke eines oder mehrerer unangenehm riechender Stoffe,
   und/oder
(b) zum Verstärken des bzw. eines oder mehrerer angenehmer Geruchseindrücke eines oder mehrerer angenehm riechender Stoffe,
umfassend folgenden Schritt:
Vermischen des bzw. der (a) angenehm und/oder (b) unangenehm riechenden Stoffe mit einer erfindungsgemäßen Mischung wie hierin beschrieben oder einer erfindungsgemäßen Riechstoffkomposition wie hierin beschrieben,
wobei die Menge an erfindungsgemäßer Mischung wie hierin beschrieben bzw. an erfindungsgemäßer Riechstoffkomposition wie hierin beschrieben ausreicht, um (a) den bzw. die angenehmen Geruchseindrücke des bzw. der angenehm riechenden Stoffe zu verstärken und/oder (b) den bzw. die unangenehmen Geruchseindrücke des bzw. der unangenehm riechenden Stoffe zu vermindern oder zu maskieren.

Für die vorstehend beschriebenen erfindungsgemäßen Aspekte gilt vorzugsweise jeweils das oben im Zusammenhang mit einer erfindungsgemäßen Mischung Gesagte entsprechend und ebenso gilt das im Zusammenhang mit den vorstehend beschriebenen erfindungsgemäßen Aspekten Gesagte entsprechend für die erfindungsgemäße Mischung. Des Weiteren sind die hierin beschriebenen Ausführungsformen, soweit technisch sinnvoll, beliebig miteinander kombinierbar.

Die vorliegende Erfindung wird anhand der nachfolgenden Beispiele näher erläutert. Sofern nichts Anderes angegeben ist, beziehen sich dabei sämtliche Angaben auf das Gewicht.

### Beispiel 1: Herstellung einer erfindungsgemäßen Mischung

In einem Rührgefäß werden 70 kg Dimethoxypropan (95 %ig) in 62 kg Aceton vorgelegt und mit 50 kg einer Ausgangsmischung versetzt, die zu 99 Gew.-% aus alpha,alpha-Cedrandiol der Formel (Illa) wie hierin beschrieben besteht, wobei die Ausgangsmischung frei ist von beta,beta-Cedrandiol der Formel (Illb), beta,alpha-Cedrandiol der Formel (lllc) und alpha,beta-Cedrandiol der Formel (Illd) wie hierin beschrieben. Anschließend wird über einen Zeitraum von 2 Stunden eine Lösung bestehend aus 53 kg Aceton und 0,167 kg technischer Schwefelsäure bei einer Temperatur von höchstens 30 °C zugegeben. Nach einer weiteren Rührzeit von 4 Stunden wird die Reaktionsmischung mit einer Aufschlämmung bestehend aus 1,6 kg kalzinierter Soda in 5 kg Wasser auf einen pH-Wert von mindestens 8 eingestellt.

Bei anschließender Destillation werden aus der Reaktionsmischung die Leichtsieder soweit entfernt, dass eine Sumpftemperatur von 95 °C nicht überschritten wird. Nach beendeter Destillation wird der Destillationsrückstand mit 38 kg Methyl-tert.-Butylether versetzt und bei einer Temperatur von etwa 35 °C für ca. 30 Minuten gerührt. Anschließend lässt man die Reaktionsmischung ruhen, bis ein klares Zwei-Phasen-Gemisch entsteht. Die wässrige Phase wird abgetrennt, und die verbleibende organische Phase mit 12 kg Wasser versetzt. Die erhaltene Mischung wird bei einer Temperatur von etwa 35 °C für ca. 30 Minuten gerührt. Anschließend lässt man die Reaktionsmischung ruhen, bis ein klares Zwei-Phasen-Gemisch entsteht. Die wässrige Phase wird abgetrennt, und bei anschließender Destillation der organischen Phase wird Methyl-tert.-Butylether soweit entfernt, dass eine Sumpftemperatur von 95 °C bei 40 mbar nicht überschritten wird. Der Destillationsrückstand wird in 100 kg Heptan aufgenommen und mit einer wässrigen ethanolischen Lösung umkristallisiert.

Das erhaltene Reaktionsprodukt ist eine erfindungsgemäße Mischung, die zu 95 Gew.-% aus der Verbindung der Formel (la) wie hierin beschrieben besteht, wobei die Mischung frei ist von Verbindungen der Formel (Ib), (Ic) und (Id) wie hierin beschrieben.

### Vergleichsbeispiel 1: Herstellung einer Mischung im Wesentlichen bestehend aus Verbindung der Formel (la) und Verbindung der Formel (Ib)

In einem Rührgefäß werden 60 kg Dimethoxypropan (95 %ig) in 62 kg Aceton vorgelegt und mit 50 kg einer Isomerenmischung zu 85 Gew.-% bestehend aus Verbindungen der Formel (III) wie hierin beschrieben (im Wesentlichen alpha,alpha-Cedrandiol der Formel (IIIa) und beta,beta-Cedrandiol der Formel (IIIb), wobei das Gewichtsverhältnis von der Verbindung der Formel (IIIa) zur Verbindung der Formel (IIIb) im Bereich von 95 : 5 bis 99,9 : 0,1 liegt, und zudem beta,alpha-Cedrandiol (Formel (Illc)) und alpha,beta-Cedrandiol (Formel (Illd)), versetzt. Weiterhin können in der Isomerenmischung bis zu 15 Gew.-%, bezogen auf das Gesamtgewicht der eingesetzten Isomerenmischung, Verbindungen der Formel (II) und (IV) bis (VI) enthalten sein. Anschließend wird über einen Zeitraum von 2 Stunden eine Lösung bestehend aus 53 kg Aceton und 0,167 kg technischer Schwefelsäure bei einer Temperatur von höchstens 30 °C zugegeben. Nach einer weiteren Rührzeit von 4 Stunden wird die Reaktionsmischung mit einer Aufschlämmung bestehend aus 1,6 kg kalzinierter Soda in 5 kg Wasser auf einen pH-Wert von mindestens 8 eingestellt.

Bei anschließender Destillation werden aus der Reaktionsmischung die Leichtsieder soweit entfernt, dass eine Sumpftemperatur von 95 °C nicht überschritten wird. Nach beendeter Destillation wird der Destillationsrückstand mit 38 kg Methyl-tert.-Butylether versetzt und bei einer Temperatur von etwa 35 °C für ca. 30 Minuten gerührt. Anschließend lässt man die Reaktionsmischung ruhen, bis ein klares Zwei-Phasen-Gemisch entsteht. Die wässrige Phase wird abgetrennt, und die verbleibende organische Phase mit 12 kg Wasser versetzt. Die erhaltene Mischung wird bei einer Temperatur von etwa 35 °C für ca. 30 Minuten gerührt. Anschließend lässt man die Reaktionsmischung ruhen, bis ein klares Zwei-Phasen-Gemisch entsteht. Die wässrige Phase wird abgetrennt, und bei anschließender Destillation der organischen Phase wird Methyl-tert.-Butylether soweit entfernt, dass eine Sumpftemperatur von 95 °C bei 40 mbar nicht überschritten wird. Der Destillationsrückstand wird in 100 kg Heptan aufgenommen und mit einer wässrigen ethanolischen Lösung umkristallisiert.

Das erhaltene Reaktionsprodukt ist eine nicht erfindungsgemäße Mischung, die zu insgesamt 83 Gew.-% aus Verbindung der Formel (la) und Verbindung der Formel (Ib) wie hierin beschrieben besteht, wobei das Gewichtsverhältnis von der Verbindung der Formel (la) zur Verbindung der Formel (Ib) in der Mischung 95 : 5 beträgt.

### Beispiel 2: Parfümöle

**Tabelle 1: Reaktionsprodukt aus Vergleichsbeispiel 1 (VB) im Umfeld aldehydisch am Beispiel des folgenden Akkords:**

| Bestandteile | A (VB) | B (VB) | C (VB) | D (VB) | E (VB) | F (VB) |
|---|---|---|---|---|---|---|
| Aldehyde C11 MOA 10% | 285,00 | 285,00 | 285,00 | 285,00 | 285,00 | 285,00 |
| 2-Methyldecanal | | | | | | |
| Aldehyde C11 10% | 95,00 | 95,00 | 95,00 | 95,00 | 95,00 | 95,00 |
| Undecanal | | | | | | |
| Aldehyde C12 MNA 10% | 38,00 | 38,00 | 38,00 | 38,00 | 38,00 | 38,00 |
| 2-Methylundecanal | | | | | | |
| Farenal^{®} 10% | 76,00 | 76,00 | 76,00 | 76,00 | 76,00 | 76,00 |
| 2,6,10-Trimethyl-undec-9-enal | | | | | | |
| Florazon 10% | 209,00 | 209,00 | 209,00 | 209,00 | 209,00 | 209,00 |
| 3-(4-Ethylphenyl)-2,2-dimethylpropanal | | | | | | |
| Limonenal 10% | 95,00 | 95,00 | 95,00 | 95,00 | 95,00 | 95,00 |
| 3-(4-Methyl-1-cyclohex-3-enyl)-butanal | | | | | | |
| Mandarin Aldehyde 10% TEC | 114,00 | 114,00 | 114,00 | 114,00 | 114,00 | 114,00 |
| (E)-Dodec-2-enal | | | | | | |
| Ozonil 10% | 38,00 | 38,00 | 38,00 | 38,00 | 38,00 | 38,00 |
| Tridec-2-enenitril | | | | | | |
| Reaktionsprodukt aus **Vergleichsbeispiel 1** | - | 0,50 | 1,00 | 5,00 | 10,00 | 30,00 |
| DPG | 50,00 | 49,50 | 49,00 | 45,00 | 40,00 | 20,00 |
| Summe | 1.000,00 | 1.000,00 | 1.000,00 | 1.000,00 | 1.000,00 | 1.000,00 |

**Tabelle 2: Reaktionsprodukt aus Beispiel 1 (B) im Umfeld aldehydisch am Beispiel des folgenden Akkords:**

| Bestandteile | A (B) | B (B) | C (B) | D (B) | E (B) | F (B) |
|---|---|---|---|---|---|---|
| Aldehyde C11 MOA 10% | 285,00 | 285,00 | 285,00 | 285,00 | 285,00 | 285,00 |
| 2-Methyldecanal | | | | | | |
| Aldehyde C11 10% | 95,00 | 95,00 | 95,00 | 95,00 | 95,00 | 95,00 |
| Undecanal | | | | | | |
| Aldehyde C12 MNA 10% | 38,00 | 38,00 | 38,00 | 38,00 | 38,00 | 38,00 |
| 2-Methylundecanal | | | | | | |
| Farenal^{®} 10% | 76,00 | 76,00 | 76,00 | 76,00 | 76,00 | 76,00 |
| 2,6,10-Trimethyl-undec-9-enal | | | | | | |
| Florazon 10% | 209,00 | 209,00 | 209,00 | 209,00 | 209,00 | 209,00 |
| 3-(4-Ethylphenyl)-2,2-dimethylpropanal | | | | | | |
| Limonenal 10% | 95,00 | 95,00 | 95,00 | 95,00 | 95,00 | 95,00 |
| 3-(4-Methyl-1-cyclohex-3-enyl)-butanal | | | | | | |
| Mandarin Aldehyde 10% TEC | 114,00 | 114,00 | 114,00 | 114,00 | 114,00 | 114,00 |
| (E)-Dodec-2-enal | | | | | | |
| Ozonil 10% | 38,00 | 38,00 | 38,00 | 38,00 | 38,00 | 38,00 |
| Tridec-2-enenitril | | | | | | |
| Reaktionsprodukt aus **Beispiel 1** | - | 0,50 | 1,00 | 5,00 | 10,00 | 30,00 |
| DPG | 50,00 | 49,50 | 49,00 | 45,00 | 40,00 | 20,00 |
| Summe | 1.000,00 | 1.000,00 | 1.000,00 | 1.000,00 | 1.000,00 | 1.000,00 |

In kleinen Dosierungen wie in B (VB) und C (VB) ist bei Zusatz des Reaktionsproduktes aus Vergleichsbeispiel 1 eine stärkere und strahlendere Entwicklung des Duftes zu beobachten als in A (VB). In C (VB) und D (VB) wird bei Zusatz des Reaktionsproduktes aus Vergleichsbeispiel 1 zudem der typische aldehydige, fettige und metallische Charakter des Akkords hervorgehoben. In E (VB) und F (VB) überwiegt der Einfluss des Reaktionsprodukts aus Vergleichsbeispiel 1 und gibt dem Akkord einen stark ambrierten Geruch (zur Zusammensetzung von A (VB) bis F (VB) vgl. Tabelle 1).

Im Vergleich dazu führt der Zusatz der gleichen Menge an Reaktionsprodukt aus Beispiel 1 anstelle des Zusatzes des Reaktionsprodukt aus Vergleichsbeispiel 1 in B (B) bis F (B) überraschenderweise zu einer unerwarteten Verstärkung der entsprechenden für B (VB) bis F (VB) beschriebenen Effekte. Insbesondere überwiegt in D (B) schon der Einfluss des Reaktionsprodukts aus Beispiel 1 und gibt dem Akkord einen stark ambrierten Geruch. Des Weiteren erscheinen die mit dem Reaktionsprodukt aus Beispiel 1 versetzten Parfümöle strahlender und ambrierter, wobei man diese Effekte schon in der Dosierung C (B) wahrnehmen kann (zur Zusammensetzung von A (B) bis F (B) vgl. Tabelle 2).

Bei Einsatz der erfindungsgemäßen Mischungen können daher zum Erreichen der gleichen Effekte geringere Konzentrationen eingesetzt werden.

### Beispiel 3: Parfümierte Produkte

### Waschpulver:

| **Bestandteil** | **Gew.-Anteile** | | |
|---|---|---|---|
| | (1) | (2) | (3) |
| Aldehyde C11 Undecylic 10% | 16,00 | 16,00 | 16,00 |
| Aldehyde C11 Undecylenic 10% | 18,00 | 18,00 | 18,00 |
| Aldehyde C12 Lauric 10% | 14,00 | 14,00 | 14,00 |
| Aldehyde C12 MNA 10% | 12,00 | 12,00 | 12,00 |
| Hexenal Trans-2 10% | 4,00 | 4,00 | 4,00 |
| Hexenyl Acetate Cis-3 | 4,00 | 4,00 | 4,00 |
| Vertocitral | 10,00 | 10,00 | 10,00 |
| Magnolan | 130,00 | 130,00 | 130,00 |
| Mintonat | 35,00 | 35,00 | 35,00 |
| Dihydro Myrcenol | 70,00 | 70,00 | 70,00 |
| Orange Oil | 35,00 | 35,00 | 35,00 |
| Nerolione 10% | 3,50 | 3,50 | 3,50 |
| Cantryl^{®} | 3,50 | 3,50 | 3,50 |
| Hexyl Acetate | 18,00 | 18,00 | 18,00 |
| Jasmaprunat | 18,00 | 18,00 | 18,00 |
| Aldehyde C14 So-Called | 50,00 | 50,00 | 50,00 |
| Ethyl Methyl Butyrate-2 | 8,00 | 8,00 | 8,00 |
| Manzanate | 1,20 | 1,20 | 1,20 |
| Allyl Cyclohexyl Propionate | 8,00 | 8,00 | 8,00 |
| Aprifloren^{®} | 3,00 | 3,00 | 3,00 |
| Fruitate | 1,80 | 1,80 | 1,80 |
| Ethyl Linalool | 56,00 | 56,00 | 56,00 |
| Dimethyl Benzyl Carbinyl Butyrate | 7,00 | 7,00 | 7,00 |
| Rose Abs. Type Base | 30,00 | 30,00 | 30,00 |
| Rosaphen^{®} | 30,00 | 30,00 | 30,00 |
| Damascenone Total | 1,20 | 1,20 | 1,20 |
| Damascone Alpha | 1,80 | 1,80 | 1,80 |
| Benzyl Acetate | 28,00 | 28,00 | 28,00 |
| Hedione | 56,00 | 56,00 | 56,00 |
| Hexyl Cinnamic Aldehyde Alpha | 130,00 | 130,00 | 130,00 |
| Parmanyl^{®} | 3,50 | 3,50 | 3,50 |
| Isoraldeine 70 | 28,00 | 28,00 | 28,00 |
| Isoeugenyl Acetate | 3,50 | 3,50 | 3,50 |
| Agrumex HC | 50,00 | 50,00 | 50,00 |
| Ambroxide Cryst. | 1,50 | 1,50 | 1,50 |
| Reaktionsprodukt aus Vergleichsbeispiel 1 | 0,80 | - | - |
| Reaktionsprodukt aus Beispiel 1 | - | 0,40 | 0,80 |
| Dipropylene Glycol | 109,70 | 110,10 | 109,70 |
| Summe | 1.000,00 | 1.000,00 | 1.000,00 |

Das Reaktionsprodukt aus Vergleichsbeispiel 1 (Spalte (1)) unterstreicht hier die warme holzige Fondnote und unterstützt die Haftung auf der Wäsche. Im Vergleich dazu kann der gleiche Effekt mit nur der halben Konzentration an Reaktionsprodukt aus Beispiel 1 im Vergleich zum Reaktionsprodukt aus Vergleichsbeispiel 1 erreicht werden (Spalte (2)). Bei Einsatz der gleichen Menge an Reaktionsprodukt aus Beispiel 1 wie Reaktionsprodukt aus Vergleichsbeispiel 1 (Spalte (3)) wirkt die Geruchsnote strahlender, ambrierter und hochwertiger als diejenige, des in Spalte (1) beschriebenen Produktes.

### Shampoo:

| **Bestandteil** | **Gew.-Anteile** | | |
|---|---|---|---|
| | (1) | (2) | (3) |
| Hexenol Cis-3 | 2,50 | 2,50 | 2,50 |
| Galbanum Oil 10% | 5,00 | 5,00 | 5,00 |
| Magnolan | 25,00 | 25,00 | 25,00 |
| Bergamot Oil RCO | 80,00 | 80,00 | 80,00 |
| Linalyl Acetate | 120,00 | 120,00 | 120,00 |
| Lemon Oil | 80,00 | 80,00 | 80,00 |
| Neroli Base | 10,00 | 10,00 | 10,00 |
| Lavender Oil | 6,00 | 6,00 | 6,00 |
| Thyme Oil 10% | 6,00 | 6,00 | 6,00 |
| Linalool | 60,00 | 60,00 | 60,00 |
| Phenylethyl Alcohol BA Free | 20,00 | 20,00 | 20,00 |
| Vitessence^{®} Rose De Mai | 6,50 | 6,50 | 6,50 |
| Narcisse Abs. 10% | 1,30 | 1,30 | 1,30 |
| Hedione | 80,00 | 80,00 | 80,00 |
| Jasmolactone Cis 10% | 6,00 | 6,00 | 6,00 |
| Parmanyl^{®} 10% | 12,70 | 12,70 | 12,70 |
| lonone Beta | 6,00 | 6,00 | 6,00 |
| Methyl lonone Gamma Pure | 18,00 | 18,00 | 18,00 |
| Irone Alpha 10% | 12,00 | 12,00 | 12,00 |
| Benzoin Siam Resin 50% | 5,00 | 5,00 | 5,00 |
| Coumarin | 5,00 | 5,00 | 5,00 |
| Iso E Super | 180,00 | 180,00 | 180,00 |
| Cashmeran | 12,00 | 12,00 | 12,00 |
| Isobutyl Quinoline 10% | 6,00 | 6,00 | 6,00 |
| Reaktionsprodukt aus Vergleichsbeispiel 1 | 0,2 | - | - |
| Reaktionsprodukt aus Beispiel 1 | - | 0,1 | 0,2 |
| Globalide^{®} | 45,00 | 45,00 | 45,00 |
| Dipropylene Glycol | 189,80 | 189,90 | 189,80 |
| Summe | 1.000,00 | 1.000,00 | 1.000,00 |

Das Reaktionsprodukt aus Vergleichsbeispiel 1 (Spalte (1)) verstärkt hier sowohl die Kopfnote als auch die haftenden holzigen Elemente in der Fondnote. Im Vergleich dazu kann der gleiche Effekt mit nur der halben Konzentration an Reaktionsprodukt aus Beispiel 1 im Vergleich zum Reaktionsprodukt aus Vergleichsbeispiel 1 erreicht werden (Spalte (2)). Bei Einsatz der gleichen Menge an Reaktionsprodukt aus Beispiel 1 wie Reaktionsprodukt aus Vergleichsbeispiel 1 (Spalte (3)) wirkt die Geruchsnote ambrierter, pflegender und kosmetischer als diejenige, des in Spalte (1) beschriebenen Produktes.

## Patentansprüche

1. Mischung umfassend die Verbindung der Formel (la), 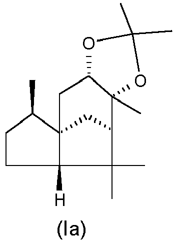 wobei die Mischung weniger als 0,01 Gew.-%, bezogen auf das Gesamtgewicht der Mischung, an Verbindung der Formel (Ib), vorzugsweise an den Verbindungen der Formeln (Ib) sowie zudem (Ic) und/oder (Id), enthält

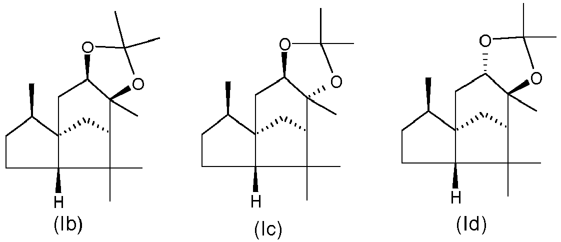

2. Mischung nach Anspruch 1, zusätzlich enthaltend eine oder mehrere 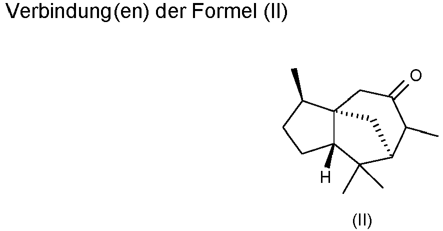 und/oder der Formel (III) 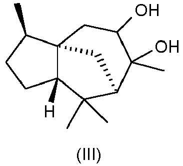 und/oder der Formel (IV) 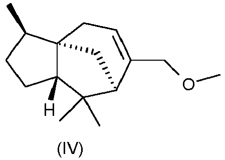 und/oder der Formel (V) 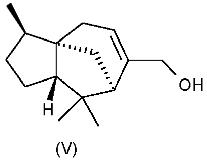 und/oder der Formel (VI)

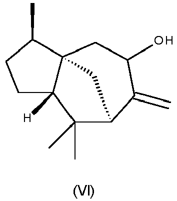

3. Mischung nach Anspruch 1 oder 2, wobei die Mischung eine oder mehrere Verbindung(en) der Formel (II) enthält, 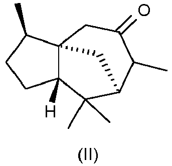
wobei das Gewichtsverhältnis der Gesamtmenge an Verbindung der Formel (la) zur Gesamtmenge an Verbindung(en) der Formel (II) in der Mischung 500 : 1 bis 3 :1, vorzugsweise 350 : 1 bis 5 : 1, besonders bevorzugt 300 : 1 bis 8 : 1, beträgt,
und/oder wobei die Mischung eine oder mehrere Verbindung(en) der Formel (III) enthält, 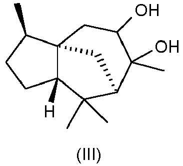
wobei das Gewichtsverhältnis der Gesamtmenge an Verbindung der Formel (la) zur Gesamtmenge an Verbindung(en) der Formel (III) in der Mischung wenigstens 5 : 1, vorzugsweise wenigstens 10 : 1, besonders bevorzugt wenigstens 15 : 1, beträgt,
und/oder wobei die Mischung eine oder mehrere Verbindung(en) der Formel (IV) enthält, 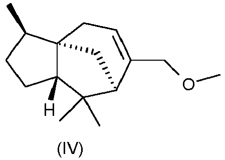
wobei das Gewichtsverhältnis der Gesamtmenge an Verbindung der Formel (la) zur Gesamtmenge an Verbindung(en) der Formel (IV) in der Mischung wenigstens 40 : 1, vorzugsweise wenigstens 50 : 1, besonders bevorzugt wenigstens 60 : 1, beträgt,
und/oder wobei die Mischung eine oder mehrere Verbindung(en) der Formel (V) enthält, 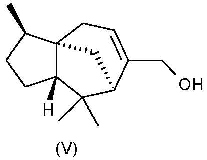
wobei das Gewichtsverhältnis der Gesamtmenge an Verbindung der Formel (Ia) zur Gesamtmenge an Verbindung(en) der Formel (V) in der Mischung wenigstens 30 : 1, vorzugsweise wenigstens 40 : 1, besonders bevorzugt wenigstens 50 : 1, beträgt,
und/oder wobei die Mischung eine oder mehrere Verbindung(en) der Formel (VI) enthält, 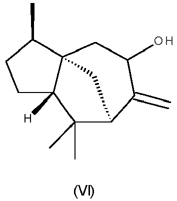
wobei das Gewichtsverhältnis der Gesamtmenge an Verbindung der Formel (la) zur Gesamtmenge an Verbindung(en) der Formel (VI) in der Mischung wenigstens 4 : 1, vorzugsweise wenigstens 6 : 1, besonders bevorzugt wenigstens 8 : 1, beträgt.

4. Verfahren zur Herstellung einer Mischung nach einem der Ansprüche 1 bis 3, bestehend aus oder umfassend folgende Schritte:
a) Bereitstellen einer Ausgangsmischung, enthaltend oder im Wesentlichen bestehend aus alpha,alpha-Cedrandiol der Formel (Illa), 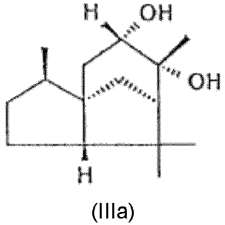 wobei die Ausgangsmischung weniger als 0,01 Gew.-%, bezogen auf das Gesamtgewicht der Ausgangsmischung, an beta,beta-Cedrandiol der Formel (Illb) enthält, 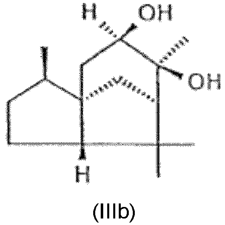 vorzugsweise wobei die Ausgangsmischung weniger als 0,01 Gew.-%, bezogen auf das Gesamtgewicht der Ausgangsmischung, an beta,beta-Cedrandiol der Formel (Illb) sowie zudem an beta,alpha-Cedrandiol der Formel (!!!c) und/oder alpha,beta-Cedrandiol der Formel (Illd) enthält, 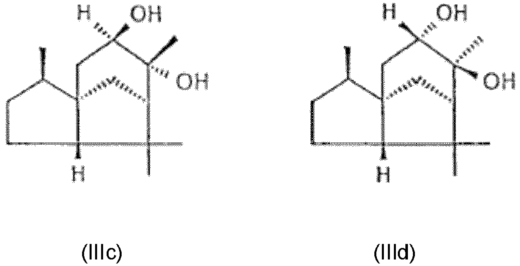 oder Bereitstellen der Verbindung alpha,alpha-Cedrandiol der Formel (Illa), 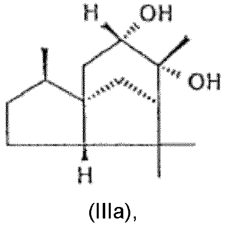
b) Umsetzen der Ausgangsmischung bzw. der Verbindung aus Schritt a) mit Dimethoxypropan,
wobei das Verfahren vorzugsweise zudem folgenden Schritt umfasst:
c) Auskristallisieren des Reaktionsprodukts aus Schritt b) aus wässriger alkoholischer Lösung.

5. Mischung nach einem der Ansprüche 1 bis 3, herstellbar durch ein Verfahren nach Anspruch 4.

6. Riechstoffkomposition, vorzugsweise Parfümöl, enthaltend oder bestehend aus einer Mischung nach einem der Ansprüche 1 bis 3 oder 5 und zudem einem oder mehreren zusätzlichen Riechstoffen,
vorzugsweise wobei der zusätzliche bzw. einer, mehrere oder sämtliche der zusätzlichen Riechstoffe ausgewählt ist bzw. sind aus der Gruppe bestehend aus 3-(4-Methyl-1-cyclohex-3-enyl)-butanal, 4-(4-Hydroxyphenyl) butan-2-on, (E)-4-(2,6,6-Trimethyl-1-cyclohex-2-enyl)but-3-en-2-on, (E)-4-(2,6,6-Trimethyl-1-cyclohex-2-enyl)but-3-en-2-on, (E)-1-(2,6,6-trimethyl-cyclohexen-1-yl)pent-1-en-3-on, (E)-4-[(1S)-1,2,6,6-tetramethylcyclohex-2-en-1-yl]-but-3-en-2-on, 1-(2,6,6-Trimethyl-1-cyclohex-2-enyl)pent-1-en-3-on, [(Z)-Hex-3-enyl] methyl carbonat, 3-[(Z)-Hex-3-enoxy]propannitril, 1-(2,3,8,8-Tetramethyl-1,3,4,5,6,7-hexa-hydronaphthalin-2-yl)ethanon, spiro[1,3-dioxolan-2,5'-(4',4',8',8'-tetramethyl-hexahydro-3',9'-methanonaphthalin)], [3R-(3α,3aβ,6β,7β,8aα)]-octa-hydro-6-methoxy-3,6,8,8-tetramethyl-1H-3a,7-methanoazulen, [3R-(3α,3aβ,7β,8aα)]-1-(2,3,4,7,8,8a-hexahydro-3,6,8,8-tetramethyl-1H-3a,7-methano-azulen-5-yl)ethan-1-on, 1-(2,2,6-Trimethyl-cyclohexyl) hexan-3-ol, 6,6-Dimethoxy-2,5,5-trimethylhex-2-en, 2,6-Dimethyloct-7-en-2-ol, 3,7-Dimethylocta-1,6-dien-3-ol, (3,7-Dimethylocta-1,6-dien-3-yl)acetat, (4-Methyl-1-propan-2-yl-1-cyclohex-2-enyl)acetat, (8E)-Cyclohexadec-8-en-1-on, 16-Oxacyclohexa-decan-1-on, 1,3,4,6,7,8-Hexahydro-4,6,6,7,8,8-hexamethyl-cyclopenta(g)-2-benzopyran, Ethoxymethoxycyclo-dodecan, 1,1,2,3,3-Pentamethyl-2,5,6,7-tetrahydroinden-4-on, 1-(2,3,8,8-Tetramethyl-1,3,4,5,6,7-hexa-hydronaphthalin-2-yl)ethanon.

7. Riechstoffkomposition nach Anspruch 6, wobei die Menge an Mischung nach einem der Ansprüche 1 bis 3 oder 5 oder die Menge an Verbindung der Formel (la) ausreicht, 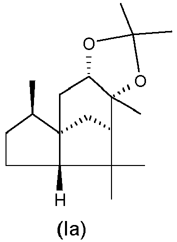
(a) um den bzw. einen oder mehrere unangenehme Geruchseindrücke eines anderen Riechstoffs in der Riechstoffkomposition zu maskieren oder zu vermindern,
und/oder
(b) um den bzw. einen oder mehrere angenehme Geruchseindrücke eines anderen Riechstoffs in der Riechstoffkomposition zu verstärken.

8. Riechstoffkomposition nach einem der Ansprüche 6 oder 7, vorzugsweise Parfümöl, wobei die Gesamtmenge an Verbindung der Formel (la) 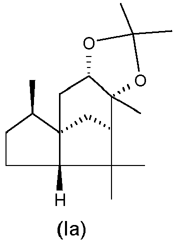 bezogen auf das Gesamtgewicht der Riechstoffkomposition 0,01 bis 10 Gew.-%, vorzugsweise 0,03 bis 5 Gew.-%, besonders bevorzugt 0,05 bis 2 Gew.-%, beträgt.

9. Parfümiertes Produkt, enthaltend eine Mischung nach einem der Ansprüche 1 bis 3 oder 5 oder vorzugsweise eine Riechstoffkomposition nach einem der Ansprüche 6 bis 8, vorzugsweise ein Parfümöl, in einer sensorisch wirksamen Menge, wobei der Anteil der Mischung bzw. der Riechstoffkomposition bezogen auf das Gesamtgewicht des Produktes vorzugsweise im Bereich von 0,01 bis 10 Gew.-%, bevorzugt 0,1 bis 5 Gew.-%, besonders bevorzugt 0,25 bis 3 Gew.-%, liegt.

10. Parfümiertes Produkt nach Anspruch 9, wobei das Produkt ausgewählt ist aus der Gruppe bestehend aus Parfüm-Extraits, Eau de Parfums, Eau de Toilettes, Rasierwässer, Eau de Colognes, Pre-shave-Produkte, Splash-Colognes, parfümierte Erfrischungstücher, saure, alkalische und neutrale Reinigungsmittel, Textilerfrischer, Bügelhilfen, flüssige Waschmittel, pulverförmige Waschmittel, Wä-schevorbehandlungsmittel, Wäscheweichspüler, Waschseifen, Waschtabletten, Desinfektionsmittel, Oberflächendesinfektionsmittel, Luftverbesserer, Aerosolsprays, Wachse und Polituren, Körperpflegemittel, Handcremes und -lotionen, Fußcremes und -lotionen, Enthaarungscremes und -lotionen, After-shave-Cremes und -lotionen, Bräunungscremes und -lotionen, Haarpflegeprodukten, Deodorantien und Antiperspirantien, Produkte der dekorativen Kosmetik, Kerzen, Lampenöle, Räucherstäbchen, Insektizide, Repellentien und Treibstoffe.

11. Verfahren zum Herstellen eines parfümierten Produktes, vorzugsweise eines Produktes nach einem der Ansprüche 9 oder 10, umfassend folgende Schritte:
i) Bereitstellen einer Mischung nach einem der Ansprüche 1 bis 3 oder 5 oder einer Riechstoffkomposition nach einem der Ansprüche 6 bis 8,
ii) Bereitstellen eines oder mehrerer weiterer Bestandteile des herzustellenden parfümierten Produktes, und
iii) Inkontaktbringen oder Mischen der in Schritt ii) bereitgestellten weiteren Bestandteile mit einer sensorisch wirksamen Menge der in Schritt i) bereitgestellten Bestandteile.

12. Verwendung einer Mischung nach einem der Ansprüche 1 bis 3 oder 5 oder einer Riechstoffkomposition nach einem der Ansprüche 6 bis 8
(a) zum Maskieren oder Vermindern des bzw. eines oder mehrerer unangenehmer Geruchseindrücke eines oder mehrerer unangenehm riechender Stoffe,
und/oder
(b) zum Verstärken des bzw. eines oder mehrerer angenehmer Geruchseindrücke eines oder mehrerer angenehm riechender Stoffe.

13. Verwendung nach Anspruch 12 in einer Zusammensetzung, die einen oder mehrere (weitere) angenehm und/oder unangenehm riechende Stoffe enthält, deren unangenehmer Geruchseindruck durch eine Mischung nach einem der Ansprüche 1 bis 3 oder 5 oder eine Riechstoffkomposition nach einem der Ansprüche 6 bis 8 maskiert oder vermindert und/oder deren angenehmer Geruchseindruck durch eine Mischung nach einem der Ansprüche 1 bis 3 oder 5 oder eine Riechstoffkomposition nach einem der Ansprüche 6 bis 8 verstärkt wird, vorzugsweise wobei diese(r) angenehm und/oder unangenehm riechende Stoff bzw. einer, mehrere oder sämtliche dieser angenehm und/oder unangenehm riechenden Stoffe ausgewählt ist bzw. sind aus der Gruppe bestehend aus 3-(4-Methyl-1-cyclohex-3-enyl)-butanal, 4-(4-Hydroxyphenyl) butan-2-on, (E)-4-(2,6,6-Trimethyl-1-cyclohex-2-enyl)but-3-en-2-on, (E)-4-(2,6,6-Trimethyl-1-cyclohex-2-enyl)but-3-en-2-on, (E)-1-(2,6,6-trimethyl-cyclohexen-1-yl)pent-1-en-3-on, (E)-4-[(1S)-1,2,6,6-tetramethylcyclohex-2-en-1-yl]-but-3-en-2-on, 1-(2,6,6-Trimethyl-1-cyclohex-2-enyl)pent-1-en-3-on, [(Z)-Hex-3-enyl] methyl carbonat, 3-[(Z)-Hex-3-enoxy]propannitril, 1-(2,3,8,8-Tetramethyl-1,3,4,5,6,7-hexa-hydronaphthalin-2-yl)ethanon, spiro[1,3-dioxolan-2,5'-(4',4',8',8'-tetramethyl-hexahydro-3',9'-methanonaphthalin)], [3R-(3α,3aβ,6β,7β,8aα)]-octa-hydro-6-methoxy-3,6,8,8-tetramethyl-1H-3a,7-methanoazulen, [3R-(3α,3aβ,7β,8aα)]-1-(2,3,4,7,8,8a-hexa-hydro-3,6,8,8-tetramethyl-1H-3a,7-methano-azulen-5-yl)ethan-1-on, 1-(2,2,6-Trimethyl-cyclohexyl)hexan-3-ol, 6,6-Dimethoxy-2,5,5-trimethylhex-2-en, 2,6-Dimethyloct-7-en-2-ol, 3,7-Dimethylocta-1,6-dien-3-ol, (3,7-Dimethylocta-1,6-dien-3-yl)acetat, (4-Methyl-1-propan-2-yl-1-cyclohex-2-enyl) acetat, (8E)-Cyclohexadec-8-en-1-on, 16-Oxacyclohexa-decan-1-on, 1,3,4,6,7,8-Hexahydro-4,6,6,7,8,8-hexamethyl-cyclopenta(g)-2-benzopyran, Ethoxymethoxycyclododecan, 1,1,2,3,3-Pentamethyl-2,5,6,7-tetrahydroinden-4-on, 1-(2,3,8,8-Tetramethyl-1,3,4,5,6,7-hexa-hydronaphthalin-2-yl)ethanon.

14. Verwendung nach Anspruch 12 oder 13, zum Verstärken des bzw. eines oder mehrerer Geruchseindrücke ausgewählt aus der Gruppe bestehend aus den Geruchsnoten blumig, vorzugsweise Maiglöckchen, Amber, holzig, Moschus, Veilchen, Citrus und aldehydig.

15. Verfahren
(a) zum Maskieren oder Vermindern des bzw. eines oder mehrerer unangenehmer Geruchseindrücke eines oder mehrerer unangenehm riechender Stoffe,
und/oder
(b) zum Verstärken des bzw. eines oder mehrerer angenehmer Geruchseindrücke eines oder mehrerer angenehm riechender Stoffe,
umfassend folgenden Schritt:
Vermischen des bzw. der (a) angenehm und/oder (b) unangenehm riechenden Stoffe mit einer Mischung nach einem der Ansprüche 1 bis 3 oder 5 oder einer Riechstoffkomposition nach einem der Ansprüche 6 bis 8,
wobei die Menge an Mischung nach einem der Ansprüche 1 bis 3 oder 5 bzw. an Riechstoffkomposition nach einem der Ansprüche 6 bis 8 ausreicht, um (a) den bzw.
die angenehmen Geruchseindrücke des bzw. der angenehm riechenden Stoffe zu verstärken und/oder (b) den bzw. die unangenehmen Geruchseindrücke des bzw. der unangenehm riechenden Stoffe zu vermindern oder zu maskieren.

## Claims

1. Mixture comprising the compound of formula (Ia), 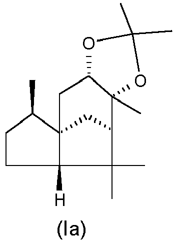 wherein the mixture contains less than 0.01 wt.-%, based on the total weight of the mixture, of compound of formula (Ib), preferably of compounds of formulae (lb) and also (Ic) and/or (Id)

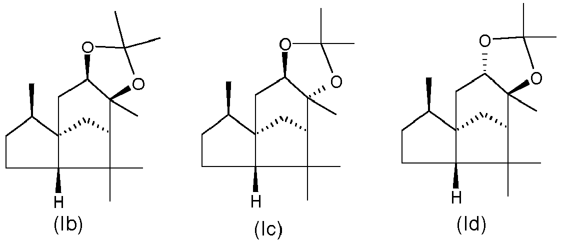

2. Mixture according to claim 1, additionally containing one or more compound(s) of formula (II) 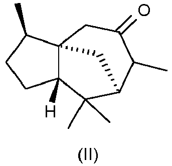 and/or of formula (III) 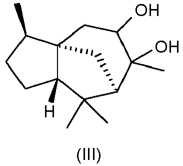 and/or of formula (IV) 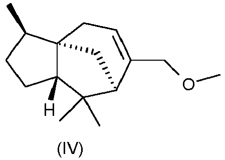 and/or the formula (V) 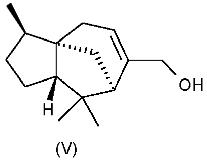 and/or of formula (VI)

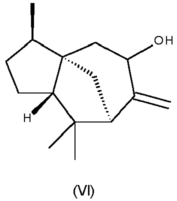

3. Mixture according to claim 1 or 2, wherein the mixture contains one or more compound(s) of formula (II), 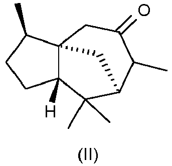
wherein the weight ratio of the total amount of compound of formula (la) to the total amount of compound(s) of formula (II) in the mixture is 500 : 1 to 3 :1, preferably 350 : 1 to 5 : 1, more preferably 300 : 1 to 8 : 1,
and/or wherein the mixture contains one or more compound(s) of formula (III), 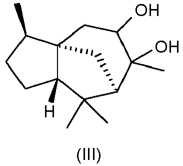
wherein the weight ratio of the total amount of compound of formula (la) to the total amount of compound(s) of formula (III) in the mixture is at least 5 : 1, preferably at least 10 : 1, more preferably at least 15:1,
and/or wherein the mixture contains one or more compound(s) of formula (IV), 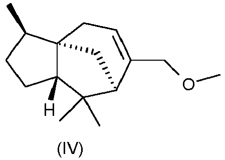
wherein the weight ratio of the total amount of compound of formula (la) to the total amount of compound(s) of formula (IV) in the mixture is at least 40 : 1, preferably at least 50 : 1, more preferably at least 60 : 1,
and/or wherein the mixture contains one or more compound(s) of formula (V), 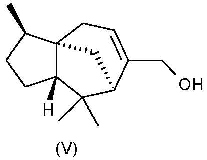
wherein the weight ratio of the total amount of compound of formula (la) to the total amount of compound(s) of formula (V) in the mixture is at least 30 : 1, preferably at least 40 : 1, more preferably at least 50 : 1,
and/or wherein the mixture comprises one or more compound(s) of formula (VI), 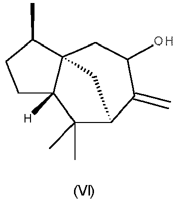
wherein the weight ratio of the total amount of compound of formula (la) to the total amount of compound(s) of formula (VI) in the mixture is at least 4:1, preferably at least 6:1, more preferably at least 8:1.

4. Process for preparing a mixture according to any one of claims 1 to 3, consisting of or comprising the following steps:
(a) Providing a starting mixture, containing or consisting essentially of alpha,alpha-cedranediol of formula (Illa), 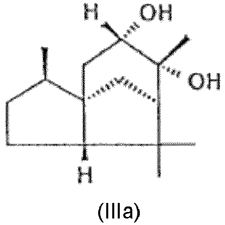 wherein the starting mixture contains less than 0.01 wt.-%, based on the total weight of the starting mixture, of beta,beta-cedranediol of formula (Illb), 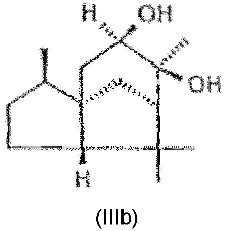 preferably wherein the starting mixture containing less than 0.01 wt.-%, based on the total weight of the starting mixture, of beta,beta-cedranediol of the formula (Illb) and also of beta,alpha-cedranediol of the formula (Illc) and/or alpha,beta-cedranediol of the formula (Illd), 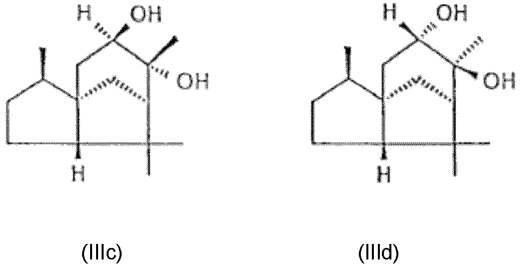 or providing the compound alpha,alpha-cedranediol of formula (Illa), 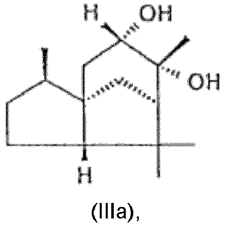
b) reacting the starting mixture or compound from step a) with dimethoxypropane,
wherein the method preferably further comprises the step of:
c) crystallizing the reaction product from step b) from aqueous alcoholic solution.

5. Mixture according to any one of claims 1 to 3, producible by a process according to claim 4.

6. Fragrance substance composition, preferably perfume oil, containing or consisting of a mixture according to any one of claims 1 to 3 or 5 and furthermore one or more additional fragrance substances,
preferably wherein the additional or one, more or all of the additional fragrance substances is/are selected from the group consisting of 3-(4-methyl-1-cyclohex-3-enyl)-butanal, 4-(4-hydroxyphenyl)butan-2-one, (E)-4-(2,6,6-trimethyl-1-cyclohex-2-enyl)but-3-en-2-one, (E)-4-(2,6,6-trimethyl-1-cyclohex-2-enyl)but-3-en-2-one, (E)-1-(2,6,6-trimethyl-cyclohexen-1-yl)pent-1-en-3-one, (E)-4-[(1S)-1,2,6,6-tetramethyl-cyclohex-2-en-1-yl]-but-3-en-2-one, 1-(2,6,6-trimethyl-1-cyclohex-2-enyl)pent-1-en-3-one, [(Z)-hex-3-enyl] methyl carbonate, 3-[(Z)-hex-3-enoxy]propanenitrile, 1-(2,3,8,8-tetramethyl-1,3,4,5,6,7-hexa-hydronaphthalen-2-yl)ethanone, spiro[1,3-dioxolane-2,5'-(4',4',8',8'-tetramethyl-hexahydro-3',9'-methanonaphthalene)], [3R-(3α,3aβ,6β,7β,8aα)]-octa-hydro-6-methoxy-3,6,8,8-tetramethyl-1H-3a,7-methano-azulene, [3R-(3α,3aβ,7β,8aα)]-1-(2,3,4,7,8,8a-hexahydro-3,6,8,8-tetramethyl-1H-3a, 7-methano-azulene-5-yl)ethan-1-one, 1-(2,2,6-trimethyl-cyclohexyl) hexan-3-ol, 6,6-dimethoxy-2,5,5-trimethylhex-2-ene, 2,6-dimethyloct-7-en-2-ol, 3,7-dimethylocta-1,6-dien-3-ol, (3,7-dimethylocta-1,6-dien-3-yl)acetate, (4-methyl-1-propan-2-yl-1-cyclohex-2-enyl)acetate, (8E)-cyclohexadec-8-en-1-one, 16-oxacyclohexa-decan-1-one, 1,3,4,6,7,8-hexahydro-4,6,6,7,8,8-hexamethyl-cyclopenta(g)-2-benzopyran, Ethoxymethoxycyclo-dodecane, 1,1,2,3,3-pentamethyl-2,5,6,7-tetrahydroinden-4-one, 1-(2,3,8,8-tetramethyl-1,3,4,5,6,7-hexahydronaphthalen-2-yl)ethanone.

7. Fragrance substance composition according to claim 6, wherein the amount of mixture according to any one of claims 1 to 3 or 5 or the amount of compound of formula (la) is sufficient, 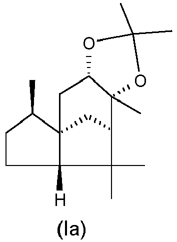
(a) to mask or reduce the or one or more unpleasant olfactory impression(s) of another fragrance substance in the fragrance substance composition,
and/or
(b) to enhance the or one or more pleasant olfactory impression(s) of another fragrance substance in the fragrance substance composition.

8. Fragrance substance composition according to any one of claims 6 or 7, preferably perfume oil, wherein the total amount of compound of formula (la) 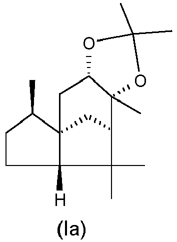 based on the total weight of the fragrance substance composition is 0.01 to 10 wt.-%, preferably 0.03 to 5 wt.-%, particularly preferably 0.05 to 2 wt.-%.

9. Perfumed product containing a mixture according to any one of claims 1 to 3 or 5 or preferably a fragrance substance composition according to any one of claims 6 to 8, preferably a perfume oil, in a sensorially effective amount, wherein the proportion of the mixture or the fragrance substance composition relative to the total weight of the product is preferably in the range from 0.01 to 10 wt.-%, preferably 0.1 to 5 wt.-%, particularly preferably 0.25 to 3 wt.-%.

10. Perfumed product according to claim 9, wherein the product is selected from the group consisting of perfume extracts, eau de parfums, eau de toilettes, shaving waters, eau de colognes, pre-shave products, splash colognes, perfumed refreshing wipes, acid, alkaline and neutral detergents, fabric fresheners, ironing aids, liquid detergents, powder detergents, laundry pre-treatment agents, laundry softeners, washing soaps, washing tablets, disinfectants, surface disinfectants, air fresheners, aerosol sprays, waxes and polishes, personal care products, hand creams and lotions, foot creams and lotions, depilatory creams and lotions, after-shave creams and lotions, tanning creams and lotions, hair care products, deodorants and antiperspirants, decorative cosmetics products, candles, lamp oils, incense sticks, insecticides, repellents, and propellants.

11. Process for preparing a perfumed product, preferably a product according to any one of claims 9 or 10, comprising the following steps:
(i) Providing a mixture according to any one of claims 1 to 3 or 5 or a fragrance substance composition according to any one of claims 6 to 8,
ii) providing one or more further ingredients of the perfumed product to be produced, and
iii) contacting or mixing the further ingredients provided in step ii) with a sensorially effective amount of the ingredients provided in step i).

12. Use of a mixture according to any one of claims 1 to 3 or 5 or a fragrance substance composition according to any one of claims 6 to 8
(a) to mask or reduce the or one or more unpleasant olfactory impression(s) of one or more unpleasant-smelling substances,
and/or
(b) to enhance the or one or more pleasant olfactory impression(s) of one or more pleasant-smelling substances.

13. Use according to claim 12 in a composition comprising one or more (further) pleasant and/or unpleasant smelling substances, the unpleasant olfactory impression of which is masked or reduced by a mixture according to any one of claims 1 to 3 or 5 or a fragrance substance composition according to any one of claims 6 to 8 and/or the pleasant olfactory impression of which is enhanced by a mixture according to any one of claims 1 to 3 or 5 or a fragrance substance composition according to any one of claims 6 to 8, preferably wherein the pleasant and/or unpleasant smelling substance or one, more or all of the pleasant and/or unpleasant smelling substance(s) is/are selected from the group consisting of 3-(4-methyl-1-cyclohex-3-enyl)-butanal, 4-(4-hydroxyphenyl)butan-2-one, (E)-4-(2,6,6-trimethyl-1-cyclohex-2-enyl)but-3-en-2-one, (E)-4-(2,6,6-trimethyl-1-cyclohex-2-enyl)but-3-en-2-one, (E)-1-(2,6,6-trimethyl-cyclohexen-1-yl)pent-1-en-3-one, (E)-4-[(1S)-1,2,6,6-tetramethyl-cyclohex-2-en-1-yl]-but-3-en-2-one, 1-(2,6,6-trimethyl-1-cyclohex-2-enyl)pent-1-en-3-one, [(Z)-hex-3-enyl] methyl carbonate, 3-[(Z)-hex-3-enoxy]propanenitrile, 1-(2,3,8,8-tetramethyl-1,3,4,5,6,7-hexa-hydronaphthalen-2-yl)ethanone, spiro[1,3-dioxolane-2,5'-(4',4',8',8'-tetramethyl-hexahydro-3',9'-methanonaphthalene)], [3R-(3α,3aβ,6β,7β,8aα)]-octa-hydro-6-methoxy-3,6,8,8-tetramethyl-1H-3a,7-methano-azulene, [3R-(3α,3aβ,7β,8aα)]-1-(2,3,4,7,8,8a-hexa-hydro-3,6,8,8-tetramethyl-1H-3a,7-methano-azulen-5-yl)ethan-1-one, 1-(2,2,6-trimethyl-cyclohexyl)hexan-3-ol, 6,6-dimethoxy-2,5,5-trimethylhex-2-ene, 2,6-dimethyloct-7-en-2-ol, 3,7-dimethylocta-1,6-dien-3-ol, (3,7-dimethylocta-1,6-dien-3-yl)acetate, (4-methyl-1-propan-2-yl-1-cyclohex-2-enyl) acetate, (8E)-cyclohexadec-8-en-1-one, 16-oxacyclohexa-decan-1-one, 1,3,4,6,7,8-hexahydro-4,6,6,7,8,8-hexamethyl-cyclopenta(g)-2-benzopyran, ethoxymethoxy-cyclododecane, 1,1,2,3,3-pentamethyl-2,5,6,7-tetrahydroinden-4-one, 1-(2,3,8,8-tetramethyl-1,3,4,5,6,7-hexa-hydronaphthalen-2-yl)ethanone.

14. Use according to claim 12 or 13, for enhancing the or one or more olfactory impressions selected from the group consisting of the olfactory notes floral, preferably lily of the valley, amber, woody, musk, violet, citrus, and aldehydic.

15. Process
(a) for masking or reducing the or one or more unpleasant olfactory impression(s) of one or more unpleasant-smelling substances,
and/or
(b) for enhancing the or one or more pleasant olfactory impression(s) of one or more pleasant-smelling substances,
comprising the following step:
Mixing the (a) pleasant and/or (b) unpleasant smelling substance(s) with a mixture according to any one of claims 1 to 3 or 5 or a fragrance substance composition according to any one of claims 6 to 8,
wherein the amount of mixture according to any one of claims 1 to 3 or 5, or of fragrance substance composition according to any one of claims 6 to 8, is sufficient to (a) enhance the pleasant olfactory impression(s) of the pleasant-smelling substance(s) and/or to (b) reduce or mask the unpleasant olfactory impression(s) of the unpleasant-smelling substance(s).

## Revendications

1. Mélange comprenant le composé de formule (la), 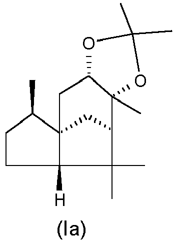 le mélange contenant moins de 0,01 % en poids, par rapport au poids total du mélange, d'un composé de formule (Ib), de préférence des composés de formules (Ib) ainsi que en sus (Ic) et/ou (Id)

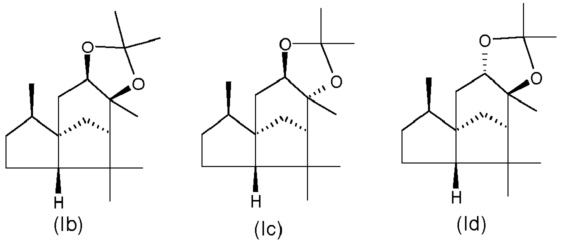

2. Mélange selon la revendication 1, contenant en outre un ou plusieurs composé(s) de formule (II) 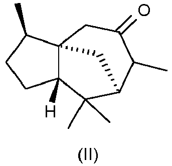 et/ou de formule (III) 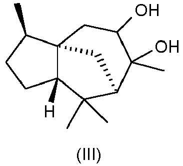 et/ou de formule (IV) 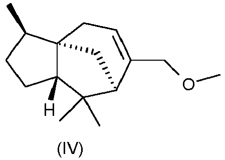 et/ou de formule (V) 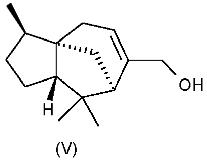 et/ou de formule (VI)

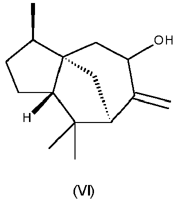

3. Mélange selon la revendication 1 ou 2, dans lequel le mélange contient un ou plusieurs composé(s) de formule (II), 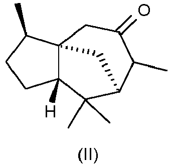
dans lequel le rapport pondéral de la quantité totale de composé de formule (la) à la quantité totale de composé(s) de formule (II) dans le mélange est de 500 : 1 à 3 : 1, de préférence de 350 : 1 à 5 : 1, de manière particulièrement préférée de 300 : 1 à 8 : 1,
et/ou dans lequel le mélange contient un ou plusieurs composé(s) de formule (III), 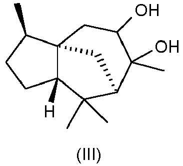
dans lequel le rapport pondéral de la quantité totale de composé de formule (la) à la quantité totale de composé(s) de formule (III) dans le mélange est d'au moins 5 : 1, de préférence d'au moins 10 : 1, de manière particulièrement préférée il est d'au moins 15 : 1,
et/ou dans lequel le mélange contient un ou plusieurs composé(s) de formule (IV), 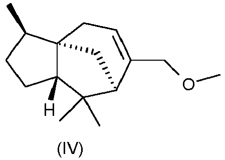
dans lequel le rapport pondéral de la quantité totale de composé de formule (la) à la quantité totale de composé(s) de formule (IV) dans le mélange est d'au moins 40 : 1, de préférence d'au moins 50 : 1, de manière particulièrement préférée il est d'au moins 60 : 1,
et/ou dans lequel le mélange contient un ou plusieurs composé(s) de formule (V), 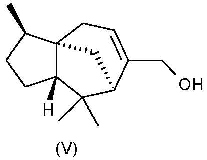
dans lequel le rapport pondéral de la quantité totale de composé de formule (la) à la quantité totale de composé(s) de formule (V) dans le mélange est d'au moins 30 : 1, de préférence d'au moins 40 : 1, de manière particulièrement préférée il est d'au moins 50 : 1,
et/ou dans lequel le mélange contient un ou plusieurs composé(s) de formule (VI), 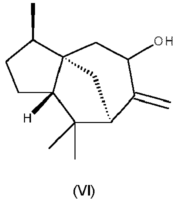
dans lequel le rapport pondéral de la quantité totale de composé de formule (la) à la quantité totale de composé(s) de formule (VI) dans le mélange est d'au moins 4 : 1, de préférence d'au moins 6 : 1, de manière particulièrement préférée il est d'au moins 8 : 1.

4. Procédé de production d'un mélange selon l'une quelconque des revendications 1 à 3, consistant en ou comprenant les étapes suivantes:
a) fournir un mélange de départ contenant ou constitué pour l'essentiel d'alpha,alpha-cédranediol de formule (IIIa), 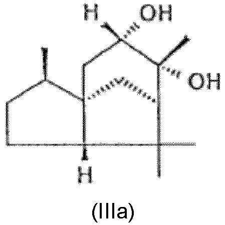 dans lequel le mélange de départ contient moins de 0,01 % en poids, par rapport au poids total du mélange de départ, de bêta,bêta-cédranediol de formule (Illb), 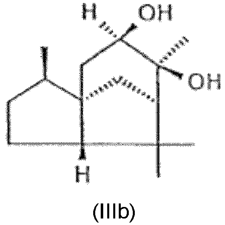 de préférence dans lequel le mélange de départ contient moins de 0,01 % en poids, par rapport au poids total du mélange de départ, de bêta,bêta-cédranediol de formule (Illb) ainsi que en sus de bêta,alpha-cédranediol de formule (Illc) et /ou d'alpha,bêta-cédranediol de formule (Illd), 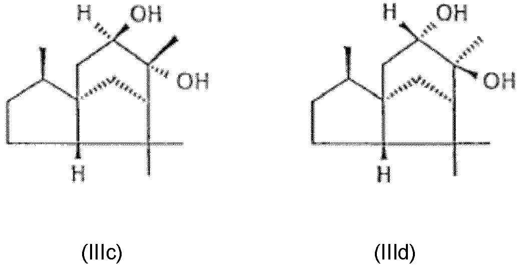 ou fournir le composé alpha,alpha-cédranediol de formule (IIIa), 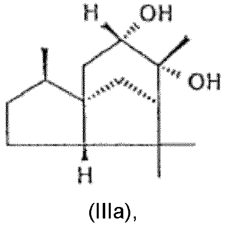
b) faire réagir le mélange de départ ou bien le composé de l'étape a) avec du diméthoxypropane,
le procédé comprenant de préférence en outre l'étape suivante consistant à:
c) faire cristalliser le produit de réaction provenant de l'étape b) à partir de solution alcoolique aqueuse.

5. Mélange selon l'une quelconque des revendications 1 à 3, apte à être préparé par un procédé selon la revendication 4.

6. Composition de substances odoriférantes, de préférence huile de parfum, contenant ou constituée d'un mélange selon l'une quelconque des revendications 1 à 3 ou 5 ainsi que, en sus, un ou plusieurs substances odoriférantes supplémentaires,
de préférence dans lequel ladite substance odoriférante supplémentaire ou bien une, plusieurs ou l'ensemble des substances odoriférantes supplémentaires est ou bien sont choisie(s) dans le groupe consistant en 3-(4-méthyl-1-cyclohex-3-ényl)butanal, 4-(4-hydroxyphényl)butane-2-one, (E)-4-(2,6,6-triméthyl-1-cyclohex-2-ényl)but-3-èn-2-one, (E)-4-(2,6,6-triméthyl-1-cyclohex-2-ényl)but-3-én-2-one, (E)-1-(2,6,6-triméthyl-cyclohexén-1-yl)pent-1-ène-3-one, (E)-4-[(1S)-1,2,6,6-tétraméthylcyclohex-2-èn-1-yl]-but-3-èn-2-one, 1-(2,6,6-triméthyl-1-cyclohex-2-ényl)pent-1-én-3-one, carbonate de [(Z)-hex-3-ényl]méthyle, 3-[(Z)-hex-3-énoxy]propanenitrile, 1-(2,3,8,8-tétraméthyl-1,3,4,5,6,7-hexahydronaphtalén-2-yl)éthanone, spiro[1,3-dioxolane-2,5'-(4',4',8',8'-tétraméthyl-hexahydro-3',9'-méthanonaphtalène)], [3R-(3α,3aβ,6β,7β,8aα)]-octa-hydro-6-méthoxy-3,6,8,8-tétraméthyl-1H-3a,7-méthanoazulène, [3R-(3α,3aβ,7β,8aα)]-1-(2,3,4,7,8,8a-hexahydro-3, 6,8,8-tétraméthyl-1H-3a,7-méthano-azulène-5-yl)éthan-1-one, 1-(2,2,6-triméthyl-cyclohexyl)hexan-3-ol, 6,6-diméthoxy-2,5,5-triméthylhex-2-ène, 2,6-diméthyloct-7-ène-2-ol, 3,7-diméthylocta-1,6-diène-3-ol, acétate de (3,7-diméthylocta-1,6-dién-3-yle), acétate de (4-méthyl-1-propan-2-yl-1-cyclohex-2-ényle), (8E)-cyclohexadéc-8-èn-1-one, 16-oxacyclohexa-décane-1-one, 1,3,4,6,7,8-hexahydro-4,6,6,7,8,8-hexaméthylcyclopenta(g)-2-benzopyrane, éthoxyméthoxycyclododécane, 1,1,2,3,3-pentaméthyl-2,5,6,7-tétrahydroindène-4-one, 1-(2,3,8,8-tétraméthyl-1,3,4,5,6,7-hexa-hydronaphtalène-2 -yl)éthanone.

7. Composition de substances odoriférantes selon la revendication 6, dans laquelle la quantité de mélange selon l'une quelconque des revendications 1 à 3 ou 5 ou la quantité de composé de formule (Ia) suffit 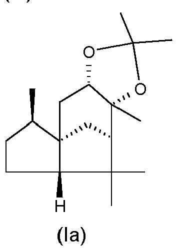
(a) pour masquer ou pour réduire la ou bien une ou plusieurs impressions olfactives désagréables d'une autre substance odoriférante dans la composition de substances odoriférantes,
et/ou
(b) pour intensifier la ou bien une ou plusieurs impressions olfactives agréables d'une autre substance odoriférante dans la composition de substances odoriférantes.

8. Composition de substances odoriférantes selon l'une quelconque des revendications 6 ou 7, de préférence huile de parfum, dans lequel la quantité totale de composé de formule (Ia) 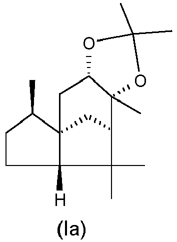 par rapport au poids total de la composition de substances odoriférantes, est de 0,01 à 10 % en poids, de préférence de 0,03 à 5 % en poids, de manière particulièrement préférée de 0,05 à 2 % en poids.

9. Produit parfumé contenant un mélange selon l'une quelconque des revendications 1 à 3 ou 5 ou, de préférence, une composition de substances odoriférantes selon l'une quelconque des revendications 6 à 8, de préférence une huile de parfum, en une quantité sensoriellement efficace, dans lequel la proportion du mélange ou bien de la composition de substances odoriférantes par rapport au poids total du produit se situe de préférence dans la plage allant de 0,01 à 10 % en poids, de préférence dans la plage allant de 0,1 à 5 % en poids, de manière particulièrement préférée dans la plage allant de 0,25 à 3 % en poids.

10. Produit parfumé selon la revendication 9, dans lequel ledit produit est choisi dans le groupe constitué par les extraits de parfum, les eaux de parfum, les eaux de toilette, les eaux après-rasage, les Eaux de Cologne, les produits de pré-rasage, les Splash Cologne, les lingettes rafraîchissantes parfumées, les agents de nettoyage acides, alcalins et neutres, les produits à rafraîchir les textiles, les aides au repassage, les détergents liquides, les détergents en poudre, les produits de prétraitement de linge, les adousissants de linge, les savons à laver, les pastilles lave-linge, les désinfectants, les désinfectants de surface, les assainisseurs d'air, les aérosols, les cires et polis, les produits de toilette, les crèmes et lotions pour les mains, les crèmes et lotions pour les pieds, les crèmes et lotions dépilatoires, les crèmes et lotions après-rasage, les crèmes et lotions de bronzage, les produits de soins des cheveux, les désodorisants et antiperspirants, les produits de la cosmétique décorative, les bougies, les huiles de lampe, les bâtonnets d'encens, les insecticides, les répulsifs et les carburants.

11. Procédé de production d'un produit parfumé, de préférence d'un produit selon l'une quelconque des revendications 9 ou 10, comprenant les étapes suivantes consistant à:
i) fournir un mélange selon l'une quelconque des revendications 1 à 3 ou 5 ou une composition de substances odoriférantes selon l'une quelconque des revendications 6 à 8,
ii) fournir un ou plusieurs autres composants du produit parfumé à produire, et
iii) mettre en contact ou mélanger les autres composants fournis à l'étape ii), avec une quantité sensoriellement efficace des composants fournis à l'étape i).

12. Utilisation d'un mélange selon l'une quelconque des revendications 1 à 3 ou 5 ou d'une composition de substances odoriférantes selon l'une quelconque des revendications 6 à 8
(a) pour masquer ou pour réduire la ou bien une ou plusieurs impressions olfactives désagréables d'une ou de plusieurs substance(s) ayant une odeur désagréable,
et/ou
(b) pour intensifier la ou bien une ou plusieurs impressions olfactives agréables d'une ou de plusieurs substance(s) ayant une odeur agréable.

13. Utilisation selon la revendication 12, dans une composition qui contient une ou plusieurs (autres) substances ayant une odeur agréable et/ou désagréable dont l'impression olfactive désagréable est masquée ou réduite par un mélange selon l'une quelconque des revendications 1 à 3 ou 5 ou une composition de substances odoriférantes selon l'une quelconque des revendications 6 à 8 et/ou dont l'impression olfactive agréable est intensifiée par un mélange selon l'une quelconque des revendications 1 à 3 ou 5 ou une composition de substances odoriférantes selon l'une quelconque des revendications 6 à 8, de préférence dans laquelle cette (ces) substance(s) ayant une odeur agréable et/ou désagréable ou bien une, plusieurs ou l'ensemble de ces substances ayant une odeur agréable et/ou désagréable est ou bien sont choisie(s) dans le groupe se composant de 3-(4-méthyl-1-cyclohex-3-ényl)-butanal, 4-(4-hydroxyphényle) butan-2-one, (E)-4-(2,6,6-triméthyl-1-cyclohex-2-ényl)but-3-én-2-one, (E)-4-(2,6,6-triméthyl-1-cyclohex-2-ényl)but-3-én-2-one, (E)-1-(2,6,6-triméthyl-cyclohexén-1-yl)pent-1-én-3-one, (E)-4-[(1S)-1,2,6,6-tétraméthylcyclohex-2-én-1-yl]-but-3-én-2-one, 1-(2,6,6-triméthyl-1-cyclohex-2-ényl)pent-1-én-3-one, [(Z)-hex-3-ényl] méthyl carbonate, 3- [(Z)-hex-3-énoxy]propannitrile, 1-(2,3,8,8-tétraméthyl-1,3,4,5,6,7-hexa-hydronaphthalin-2-yl)éthanone, spiro[1,3-dioxolan-2,5'-(4',4',8',8'-tétraméthyl-hexahydro-3',9'-méthanonaphthaline)], [3R-(3α,3aβ,6β,7β,8aα)]-octa-hydro-6-méthoxy-3,6,8,8-tétraméthyl-1H-3a,7-méthanoazulène, [3R-(3α,3aβ,7β,8aα)]-1-(2,3,4,7,8,8a-hexahydro-3,6,8,8-tétraméthyl-1H-3a,7-méthano-azulén-5-yl)éthan-1-one, 1-(2,2,6-triméthyl-cyclohexyl)hexan-3-ol, 6,6-diméthoxy-2,5,5-triméthylhex-2-ène, 2,6-diméthyloct-7-én-2-ol, 3,7-diméthylocta-1,6-dién-3-ol, acetate de (3,7-diméthylocta-1,6-dién-3-yl), acétate de (4-méthyl-1-propan-2-yl-1-cyclohex-2-ényl), (8E)-cyclohexadéc-8-én-1-one, 16-oxacyclohexa-décan-1-one, 1,3,4,6,7,8-hexahydro-4,6,6,7,8,8-hexaméthyl-cyclopenta(g)-2-benzopyrane, éthoxyméthoxycyclododécane, 1,1,2,3,3-pentaméthyl-2,5,6,7-tétrahydroindén-4-one, 1-(2,3,8,8-tétraméthyl-1,3,4,5,6,7-hexa-hydronaphthalin-2-yl)éthanone.

14. Utilisation selon la revendication 12 ou 13, pour intensifier la ou bien une ou plusieurs impressions olfactives choisies dans le groupe constitué par les notes olfactives fleuries, de préférence muguet, ambre, boisées, musc, violette, agrume et aldéhydique.

15. Procédé
(a) pour masquer ou pour réduire la ou bien une ou plusieurs impressions olfactives désagréables d'une ou de plusieurs substance(s) ayant une odeur désagréable,
et/ou
(b) pour intensifier la ou bien une ou plusieurs impressions olfactives agréables
d'une ou de plusieurs substance(s) ayant une odeur agréable.
comprenant les étapes suivantes consistant à:
mélanger la ou bien les substances (a) ayant une odeur agréable et/ou (b) ayant une odeur désagréable avec un mélange selon l'une quelconque des revendications 1 à 3 ou 5 ou une composition de substances odoriférantes selon l'une quelconque des revendications 6 à 8,
dans lequel la quantité de mélange selon l'une quelconque des revendications 1 à 3 ou 5 ou bien de composition de substances odoriférantes selon l'une quelconque des revendications 6 à 8 est suffisante pour (a) intensifier la ou bien les impressions olfactives agréables de la ou bien des substance(s) ayant une odeur agréable, et/ou pour (b) réduire ou masquer la ou bien les impressions olfactives désagréables de la ou bien des substance(s) ayant une odeur désagréable.
